# EUROPÄISCHE PATENTSCHRIFT

(11) **EP 0 728 844 B1**
(45) Veröffentlichungstag und Bekanntmachung des Hinweises auf die Patenterteilung: **08.12.1999**
(21) Anmeldenummer: 96102168.0
(22) Anmeldetag: 14.02.1996
(51) Int. Cl.: C14C 1/06, C14C 1/08

(54) **Mehrfunktionelle Lederbearbeitungsmittel**
Multifunctional leather processing compositions
Composés multifonctionnels pour la fabrication du cuir

(30) Priorität: 24.02.1995 DE 29503135 U
(43) Veröffentlichungstag der Anmeldung: 28.08.1996
(73) Patentinhaber: Röhm GmbH, 64293 Darmstadt (DE)
(72) Erfinder: Christner, Jürgen, Dr., D-64342 Seeheim-Jugenheim (DE); Wick, Gertrud, D-64291 Darmstadt (DE)

(56) Entgegenhaltungen:
- EP-A- 0 505 920
- US-A- 2 556 649

## Beschreibung

Die Erfindung betrifft ein stabiles, wässriges Flüssigmittel für die Lederherstellung in der Wasserwerkstatt in Form eines Kombinationspräparats, enthaltend eine Lösung von proteolytischen und/oder lipolytischen Enzymen, hohe Konzentrationen an Melasse, und vorzugsweise Hydrotropica sowie gegebenenfalls andere, dispergierende, schwellungsdämpfende, haarlockernde oder kalklösende Zusätze. Die Flüssigmittel dienen vor allem der Verbesserung der Rehydratisierung und Schmutzentfernung in der Weiche, der Verbesserung der Haarlockerung und der Schwellungsdämpfung im Äscher und der Verbesserung der Oberflächenreinigung der Haut in der Beize.

### Stand der Technik

Die Verfahren der Lederherstellung in der Wasserwerkstatt, nämlich die Weiche, der Äscher und die Beize, die alle der Vorbereitung der Gerbung dienen, verlangen eine Vielzahl von Behandlungsschritten mit wechselnden Zugaben von Reagentien und Zusätzen zur Flotte. Es werden Enzyme, Hydrotropika, Tenside, schwellungsdämpfende, kalkdispergierende und haarlockernde Wirkstoffe zugesetzt. Sie werden in der Regel einzeln zugegeben; es ist nicht üblich, verschiedene Reagentien in einem Mittel zu vereinigen. Dies gilt besonders für den Enzymeinsatz in der Wasserwerkstatt: Enzyme werden meistens als Einzelpräparate eingesetzt. Kombinationspräparate, die enzymatische Funktionen mit anderen, z.B. hydratisierenden Funktionen in einer Zubereitung vereinigen, haben nicht Eingang in die industrielle Praxis gefunden.

### Die Verwendung von Enzymen

Enzymatische Prozesse als Prototypen einer "sanften Technologie" werden heute auf verschiedenen Technologiefeldern bevorzugt. So gibt es nicht nur in der Lederindustrie, sondern auch in der Waschmittelindustrie, bei der Futtermittel- und Nahrungsmittelherstellung bereits bewährte, enzymatische Verfahren; eine quantitative und qualitative Ausweitung wird allgemein angestrebt. Der heutige Stand und die Zukunftsperspektiven der Enzymtechnologie sind in Ullmann, Encyclopedia of Industrial Chemistry, 5th Ed., Vol. A15, VCH (1990), Seite 390-434 beschrieben.

Zum Enzymeinsatz in der Lederverarbeitung, insbesondere in der Wasserwerkstatt, gibt es einen breiten Stand der Technik. Der gezielte Einsatz von Enzymen bei der Lederherstellung nahm seinen Anfang mit der Einführung der enzymatischen Beize durch Dr. Otto Röhm im Jahre 1907 (DE-PS 200 519). Seit dieser Zeit wurde - vor dem Hintergrund eines zunehmenden oekologischen Bewusstseins - die Anwendung von Proteasen bei verschiedenen Teiloperationen der Wasserwerkstatt vorgeschlagen und auch praktisch verwirklicht (vgl. E. Pfleiderer und R. Reiner in Biotechnology Ed. H.-J. Rehm Vol. 6b, S. 729 - 743, VCH 1988). US-A-2 556 649 erläutert die Verwendung von Melasse und Enzymen in der Lederverarbeitung.

### Die proteolytischen Enzyme

Proteolytische Enzyme werden in der Weiche, im Äscher und in der Beize eingesetzt.

### Die Weiche:

Die Häute werden in trockenem Zustand angeliefert und müssen zur weiteren Behandlung rehydratisiert (aufgeweicht), vorgereinigt und entfettet werden. Proteolytische Enzyme helfen hier auf folgende Weise:
1. Albumine und Globuline von Blutresten werden hydrolysiert und von der Oberfläche entfernt.
2. Proteoglykane, die die Kollagenfasern ummanteln, werden ebenfalls entfernt.
3. Dadurch wird die oberste Hautschicht (Epidermis) durchlässiger, Wasser und mit ihm die zugesetzten Tenside dringen schnell und tief ein. Letztere erreichen somit schnell ihren Wirkungsort, was zu einer guten Entfettung führt.
Man erkennt die Wirkung der Enzyme daran, dass die Haut schneller rehydratisiert und vollständiger entfettet wird und nach der Weiche glatter, sauberer und weicher ist.

### Das Äscherverfahren

Anschließend erfolgt die Enthaarung durch Einsatz von starken Alkalien ("Äschern") und Reduktionsmitteln, wie Sulfiden. Der Einsatz von Proteinasen unterstützt die Haarlockerung und verbessert die Glätte und Reinheit der geäscherten Haut. Die Neutralisation ("Entkälken") wird mit organischen Säuren durchgeführt.

### Die Beize:

Dieser Behandlungsschritt beinhaltet eine intensive Oberflächenreinigung und soll auch für gute Weichheit und Elastizität sorgen. Enzyme erfüllen hier folgende Funktionen:
1. Nicht lederbildende Proteine werden weggereinigt, Reste von Haarwurzeln und Fett entfernt.
2. Elastin im Narben wird teilweise abgebaut, es erhöht sich die Weichheit.
3. Auch das Kollagengerüst wird durch Spaltung im Telopeptidbereich der Fasern leicht gelockert. Das Leder wird weich und zeigt eine verbesserte Grundlockerung.

Die Häute und Felle sind nun für die Weiterbehandlung vorbereitet: als nächster Schritt erfolgt die Gerbung. Die durch eine gelungene Weiche und Beize erzeugte reine und fehlerfreie Oberfläche erlaubt zuletzt auch eine gleichmäßige Anfärbung.
Bei den in obigen Prozessen in der Wasserwerkstatt eingesetzten Proteinasen handelt es sich um neutrale (E.C.3.4.24) und insbesondere alkalische Proteasen (E.C.3.4.21) [vgl. Kirk-Othmer, 3rd. Ed. Seite 199 - 199 - 202, J. Wiley Ullmann's Encyclopedia of Industrial Chemistry, 5th Ed., Vol. A9, Seite 409 - 414, VCH 1987; L. Keay in "Process Biochemistry" Seite 17 - 21 (1971)].
Im einzelnen sind dies alkalische Bacillus-Proteasen, die ihr Wirkungsoptimum im pH-Bereich 8.5 bis 13 entfalten und die zumeist dem Serin-Typ angehören, und alkalische Pilzproteasen. Genannt seien vor allem die Proteasen aus Bacillus-Stämmen wie B. subtilis, B. licheniformis, B. firmus, B. alcalophilus, B. polymixa, B. mesentericus, ferner Streptomyces-Stämme wie S. alcalophilus. Die günstigste Arbeitstemperatur für alkalische Bakterien-Proteasen liegt im Allgemeinen bei 40-60 °C, bei Pilzproteasen eher bei 20-40 °C. Als alkalische Pilzproteasen seien genannt: solche aus Aspergillus-Stämmen wie A. oryzae, aus Penicillium-Stämmen wie P.
cyanofulvum oder aus Paecilomyces persicinus u.ä. Die Aktivität der alkalischen Pilzproteasen liegt vorwiegend im pH-Bereich 8,0 - 11,0. Neutrale Proteasen mit Wirkungsoptimum im Bereich von pH 6,0 - 9,0. können ebenfalls verwendet werden, auch wenn sie im hochalkalischen Bereich weniger wirken. Dazu gehören neutrale Bakterienproteasen, die in der Regel zu den Metalloenzymen gehören, beispielsweise neutrale Bacillus-Proteasen, wie von B. subtilis, B. licheniformis, B. natto und B. polymixa, Pseudomonas-Proteasen, Streptomyces-Proteasen, aber auch Pilzproteasen wie Aspergillus-Proteasen aus A. oryzae, A. parasiticus und Penicillium-Proteinasen, wie P. glaucum. Neutrale Bakterienproteasen entfalten ihre Aktivität optimal bei Arbeitstemperaturen von 20-50 °C, wogegen die günstigste Arbeitstemperatur für neutrale Pilzproteasen bei 35-40 °C liegt. Die proteolytische Wirksamkeit der Enzyme wird gebräuchlicherweise nach der Anson-Haemoglobin-Methode (M.L. Anson, J. Gen. Physiol, 22, Seite 79ff, 1939) bzw. nach der Löhlein-Volhard-Methode (modifiziert nach TEGEWA in Leder 22, Seite 121 - 126, 1971) bestimmt. Dabei entspricht eine Löhlein-Volhard-Einheit (LVE) unter den Testbedingungen (1 Stunde, 37°C, pH=8.3) einer Enzymmenge, die in 20 ml Casein-Filtrat einen Anstieg an Hydrolyseprodukt entsprechend einem Äquivalent von 5.75 x 10-³ ml 0.1 n NaOH hervorruft.

### Andere Enzyme und Enzymkombinationen

Der von Dr. Otto Röhm in die Ledertechnologie eingeführte pankreatische Enzymkomplex ist als Enzymkombinations-Präparat anzusehen, denn er enthält bereits mehrere enzymatische Aktivitäten, nämlich Amylasen, Lipasen, endo- und exo-Proteinasen, wobei freilich die tryptische Aktivität der letzteren in der Anwendung dominiert.
Amylasen, insbesondere in Kombination mit Proteasen haben Eingang in das Beiz-Verfahren der Wasserwerkstatt gefunden (US-A 4 273 876). Die gleichzeitige Anwendung von Lipase und Amylase (in Form des Pankreatins) in Anwesenheit von Desoxycholsäure ist aus dem ungar. Patent 3325 (Chem. Abstr. 77, 7341 k) bekannt.
In jüngster Zeit wird ein enzymatisch unterstütztes Weichverfahren für Häute und Felle empfohlen, bei dem die Weichflotten Lipasen mit einem Wirkungsoptimum im pH-Bereich 9 bis 11, Proteasen mit Wirksamkeit im pH-Bereich 9 - 11 und grenzflächenaktive Agentien enthalten, wobei der pH-Wert der Weichflotte im Bereich 9 - 11 liegt (vgl. deutsche Patentanmeldung P 39 22 748.0).Demnach sind auch Lipasen wirksam. Als besonders geeignet werden dabei aus Aspergillus-Arten gewonnene und speziell gewisse genetisch veränderte Stämme befunden, beispielsweise eine alkalische Lipase aus einem durch Rekombination gewonnenen Aspergillus-oryzae-Stamm mit ausgeprägtem Aktivitäts-Optimum zwischen pH 9 und 11 (beschrieben in USP 5 082 585). Sie entspricht der im Handel unter der Bezeichnung "LIPOLASE 100 T®" befindlichen Lipase (NOVO INDUSTRI A/S, DK 2880 Bagsvaerd). Andere in Frage kommende Lipasen stammen z.B. aus Rhizopus, wie Rh. javanicus, aus Mucor, wie M. mihei oder M. javanicus, aus Pseudomonas, wie Ps. fluorescens oder aus Aspergillus niger.
In herkömmlicher Weise wird die Aktivitätsbestimmung von Lipasen mit Triacetin und Tributyrin als Substrat durchgeführt (Vgl. M. Sémériva et al., Biochemistry 10, Seite 2143ff, 1971), ferner mit Olivenöl (Vgl. Bruno Stellmach, Bestimmungsmethoden Enzyme für Pharmazie, Lebensmittelchemie, Technik, Biochemie, Biologie, Medizin, Steinkopf-Verlag. 1988, Seite 169f: Lipase nach FIP, Einheit = FIP/g). Soweit die fettspaltende Aktivität im Sauren gemessen werden soll, wird sie mit Tributyrin als Substrat analysiert. (Einheit = LCA/g). Standardbedingungen sind 40°C, pH = 5,5 (Vgl. M. Sémériva, oben zitierte Literaturstelle). Für die Zwecke der vorliegenden Erfindung wird die Lipaseaktivität vorwiegend nach FIP (FIP/g) angegeben, wobei bei pH 9,0 und 37 °C gemessen wird. In der DE-A 41 09 826 wird das Prinzip der gleichzeitigen Verwendung von Proteinasen und Lipasen im alkalischen pH-Bereich auf die Teiloperationen Äscher und Beize angewandt. Auch hier ist es gerade die Kombination der beiden Aktivitäten, die besonders wirkungsvoll ist. Die beiden Enzyme werden einzeln zugesetzt, ein fertiges Kombinationspräparat, das beide Aktivitäten vereinigt, wird aus verständlichen Gründen nicht beschrieben, muss doch bei einer derartigen Kombination mit einer "Kannibalisierung" der Enzyme gerechnet werden.

### Die Verwendung von Melasse

Die Verwendung von Melasse bei der Lederverarbeitung ist bekannt. Melasse kann in geringen Konzentrationen bei allen Operationen in der Wasserwerkstatt zugesetzt werden. Besonders wertvoll ist ihr Zusatz beim Entkälken, da sie die Löslichkeit des Kalkhydrats in der Flotte deutlich verbessert und so die vollständige Entfernung von Kalkresten begünstigt. In der "Bibliothek des Leders" Band 2, herausgegeben von H. Herfeld (1989), Seite 115, wird festgestellt, dass sich in einer 1 %igen Zuckerlösung fast viermal soviel Kalk löst wie in reinem Wasser.

### Die Verwendung von Hydrotropika in der Lederverarbeitung

Unter Hydrotropie versteht man das Phänomen, dass eine schwerlösliche Substanz in Gegenwart einer zweiten Komponente, die selbst kein Lösungsmittel darstellt, wasserlöslich wird. Substanzen, die eine derartige Löslichkeitsverbesserung bewirken, werden als Hydrotropika bezeichnet. Sie wirken als Löslichkeitsvermittler mit unterschiedlichen Wirkungsmechanismen. Dementsprechend ist ihre chemische Zusammensetzung ganz unterschiedlich. In F. Stather, "Gerbereichemie und Gerbereitechnologie", Akademieverlag Berlin (1951), Seite 70 und 71, wird zwischen Nichtelektrolyten und Elektrolyten unterschieden. Zu ersteren zählen organische Aminoverbindungen wie Harnstoff, Thioharnstoff, Formamid, Acetamid usw. Zu letzteren zählen Sulfonsäuren und Carbonsäuren der aromatischen Reihe, aber auch der aliphatischen Reihe, insbesondere deren Salze. Auch anorganische Neutralsalze wie Rhodanide oder auch Calciumchlorid besitzen - entsprechend ihrer Stellung in der Hoffmeister'schen Reihe - hydrotrope Wirkung. Hydrotropika bewirken bei Proteinen, wie z.B. dem Kollagengerüst der Haut, eine Aufspaltung der Wasserstoffbrücken zwischen den Peptidketten und somit eine Quellung, die im Falle des Kollagens vor allem den Enzymangriff erleichtert, aber auch die Ausspülbarkeit verbessert (vgl. "Bibliothek des Leders" Band 2, herausgegeben von H. Herfeld (1989), Seite 63, und Y. Nozaki, Ch. Tanford in J. Biol. Chem., 238( (1963), Seite 4075-4081).
Im Äscher wirken Hydrotropika erkennbar haarlockernd und grundlockernd.
Die Verwendung von Hydrotropika bei der enzymatischen Hydrolyse von verschiedenen löslichen und unlöslichen Proteinen ist in mehreren Patenten beschrieben. Hydrotropika, insbesondere Harnstoff, erleichtern den proteolytischen Angriff, indem sie das zu hydrolysierende Protein denaturieren. Das deutsche Patent DE-P 2 643 012 beschreibt die proteolytische Hydrolyse von Maschinenleimleder in Gegenwart von Harnstoff, das DE-P 2 705 669 die Hydrolyse von Wolle und Haaren, das DE-P 2 756 739 die von Fleischabfällen, das DE-P 2 842 918 die von Proteinen aus dem Blut. In allen Schutzrechten wird bewusst der Gehalt an Harnstoff im Hydrolyseansatz auf < 1 Mol/l - bevorzugt < 0.1 Mol/l - beschränkt, um zu vermeiden, dass das Enzymprotein selbst denaturiert wird und seine Aktivität verliert. Der Schwellenwert für eine wirkungsmäßige Beeinträchtigung der Proteinasaktivität wird also oberhalb von 1 Mol/l Harnstoff angesetzt. Somit besteht ein beträchtlicher Vorbehalt, Harnstoff in höherer Konzentration einem Flüssigpräparat, das Enzyme enthält, zuzusetzen. Enzymatische Flüssigpräparate, die Harnstoff oder andere Hydrotropika enthalten, sind nicht bekannt.
Gleiches gilt für die deutschen Patentschrift 2 813 075. Hier wird ein enzymatisches Äscherverfahren beschrieben, bei dem zur Flotte neben alkalischer Proteinase auch Harnstoff oder Guanidinhydrochlorid zugesetzt wird. Der Gehalt an Hydrotropikum in der Flotte liegt unter 1 %. Es wird getrennt vom Enzympräparat zugegeben.

Andere Zusätze bei der Lederbearbeitung in der Wasserwerkstatt Dazu zählen in erster Linie grenzflächenaktive Substanzen, z.B. die an sich üblichen Emulgatoren. Sie sollen das an der Haut haftende Fett dispergieren und auf diese Weise die Hautoberfläche reinigen. Der diesbezügliche Stand der Technik ist z.B. in der europäischen Patentanmeldung EPA 0 505 920 ausführlich beschrieben. Es werden nicht-ionogene Emulgatoren, wie Polyglykolderivate und Glycerinderivate genannt, ferner anionische Emulgatoren wie Alkyl- oder Aryl-Sulfate und -Sulfonate, sowie Aminsalze und quaternäre Ammoniumsalze. Ihnen allen ist ein HLB-Wert von 8-18, vorzugsweise von 9-15, speziell von 12-15 gemeinsam. Auch Kombinationen von verschiedenen Emulgatortypen sind in obiger EPA beschrieben.

Andere Zusätze zur Flotte bei der Lederbearbeitung in der Wasserwerkstatt sind kalkdispergierende oder kalklösende Mittel, auch Sequestriermittel genannt, die der Oberflächenreinigung der Haut von unerwünschten Ablagerungen dienen bzw. die Bildung von Kalkseifen verhindern sollen. Sequestriermittel sind z.B. Polyphosphate, Polyphosphonate, Polycarboxylate, Ethylendiamintetraessigsäure (EDTA), Nitrilotriessigsäure, Diethylentriaminopentaessigsäure und Salze der letzteren.

Haarlockernde Mittel werden beim Verarbeitungsschritt des Äscherns ebenfalls der Flotte zugesetzt. Neben alkalisierenden Zusätzen sind es vor allem Thioverbindungen wie Natrium-Mercaptoethanol oder hydroxyfunktionelle Amine wie Mono-, Di-, oder Tri-Ethanolamin. Speziell die letzteren besitzen außerdem eine ausgeprägte schwellungsdämpfende Wirkung, das heißt, die Häute zeigen bei Alkalieinwirkung im Äscher weniger Schwellung und dadurch weniger Narbenzug.
Alle genannten Zusätze zur Flotte bei der Lederverarbeitung sind Stand der Technik; sie werden einzeln der Flotte zugesetzt. Kombinationspräparate mit Enzymen sind nicht üblich.

### Die Stabilisierung von Enzympräparaten

Die Verwendung von Melasse als Stabilisierungsmittel für flüssige Enzympräparate ist nicht bekannt. Gegen die Verwendung von Melasse in einem Enzympräparat für die Lebensmitteltechnologie spricht allein schon der ästhetische Aspekt, nämlich die ausnehmend dunkle Farbe und die damit verbundenen Farbveränderungen im behandelten Produkt. Auch die nicht standardisierbare Zusammensetzung und vor allem die in der Melasse enthaltenen undefinierten thermischen Abbauprodukte,die aktivitätsmindernd wirken könnten, halten den Fachmann von einer Verwendung ab. Dagegen wird Melasse häufig zur Fermentation von Mikroorganismen als C-Quelle verwendet. M. Bekers und A. Upit in Mikrobiologiya, 41 (5), Seite 830-833 (1972) berichten von einem Stabilisierungseffekt von mit Melasse fermentierter Hefe auf ihre Viabilität als Trockenprodukt. In USP 4 201 564 wird Melasse als C-Quelle und somit als Stabilisator für ein gutes, kontinuierliches Wachstum von Bodenbakterien einem Düngemittel zugesetzt. Der Stand der Technik legt die Verwendung von Melasse in einem flüssigen Enzympräparat keinesfalls nahe.

Dagegen ist die Verwendung von verschieden Kohlenhydraten und anderen Polyolen definierter Zusammensetzung als Stabilisierungsmittel in flüssigen Enzympräparaten Stand der Technik. So beschreibt die europäische Patentanmeldung EP-A 74237 die Verwendung von Sorbit zur Stabilisierung von Lactaselösungen, das US-Patent 4 011 169 ganz allgemein die Verwendung von Polysacchariden für die Formulierung von Enzympräparaten, das US-Patent 3 133 001 nennt hierfür unter anderem Saccharose, Lactose und Maltose. In dem japanischen Patent 262 339 werden auch Alkohole zu Stabilisierung von Flüssigpräparaten, insbesondere von Proteinasen vorgeschlagen. Die Verwendung von gelösten Kohlenhydraten als Trägerflüssigkeit in enzymatischen Lederbehandlungsmitteln wurde bisher nicht beschrieben. Enzymatische Präparate, in denen Kohlenhydrate mit anderen Zusätzen wie Hydrotropika, Sequestriermitteln, Tensiden oden haarlockernden Mitteln kombiniert werden, sind nicht bekannt. In dem schweizer Patent 677 798 wird schließlich eine Flüssigformulierung von Enzymen zur technischen Anwendung, beispielsweise in der Lederindustrie, beansprucht. Die hier beschriebenen Präparate enthalten im wesentlichen wasserfreie, organische Flüssigkeiten und anorganische, pulverförmige Dispergatoren.

### Aufgabe

Enzympräparate werden, wenn es die jeweilige Anwendung erlaubt, bevorzugt in wässriger, flüssiger Form angeboten. Die flüssige Form entspricht dem Standardmilieu der Enzymreaktion, dem wässrigen Medium. Daher können wässrige, flüssige Enzympräparate schnell und direkt appliziert werden. Es entfällt - im Vergleich zu lyophilisierten Enzympräparaten - der Lösungsvorgang, der langwierig sein kann. Auch allergische Kontaktreaktionen, die z.B. bei Anwendung von lyophilisierten Enzympräparaten (insbesondere wenn sie Staub enthalten) auftreten können, sind leicht auszuschließen. Vorteilhaft sind flüssige Enzympräparate auch bei kontinuierlicher Enzymdosierung. Aufgabe der vorliegenden Erfindung ist es daher, ein flüssiges Enzympräparat zu konzipieren.

Andererseits sieht man sich bei der Verwendung von flüssigen Enzympräparaten einer Reihe von Problemen gegenüber. An erster Stelle steht die Stabilität solcher Präparate, und zwar in zweifacher Hinsicht:

### 1. Mikrobiologische Stabilität:

Ein Enzympräparat, das eine Standzeit von z.B. einem Jahr und länger haben soll, darf in dieser Zeit nicht mikrobiell befallen werden. Diese Gefahr ist durch die Zusammensetzung des Präparates gegeben, da es meist die notwendigen Komponenten einer Nährlösung für Mikroorganismen enthält. Mikrobielles Wachstum kann durch verschiedene Konservierungsmittel verhindert werden. Es genügen sehr geringe Zusatzmengen, in der Regel <1%. Eine Zusammenstellung von Konservierungsmittel nach dem Stand der Technik findet sich in K. H. Wallhäußer, Sterilisation, Desinfektion, Konservierung, Georg Thieme Verlag 1978, Seite 380. Konservierungsstoffe sind kein absolut sicheres Mittel zur Abwehr jedes mikrobiellen Wachstums, zudem sind sie oft enzymschädigend. Daher wird die Alternative für eine mikrobielle Stabilisierung, eine möglichst niedrige Wasseraktivität im Präparat, meist bevorzugt. Je geringer der Wassergehalt, desto geringer ist - bedingt durch den hohen osmotischen Druck - die Gefahr eines mikrobiellen Wachstums. Daher formuliert man Enzympräparate meist so, dass sie hohe Konzentrationen wasserlöslicher Verbindungen aller Art enthalten: Salze, Kohlenhydrate wie z.B. Zucker, und andere Polyhydroxyverbindungen wie z.B. Glycerin.

### 2. Aktivitätskonstanz:

Enzyme unterliegen in wässriger Lösung der Beeinflussung durch die übrigen vorhandenen Bestandteile des Mediums, wie Säuren, Basen, Salze, oberflächenaktive und komplexaktive Bestandteile, andere Makromoleküle und vor allem andere Enzyme. Diese Bestandteile können sowohl stabilisierend, als auch destabilisierend wirken. Die Mechanismen der Destabilisierung sind komplex, sie können thermischer, chemischer, proteolytischer Natur sein. Es kann auch keine allgemeine Aussage getroffen werde, welche der in der Literatur genannten Stabilisierungsmittel im jeweiligen Anwendungsfall wirken und welche nicht. Ein Mittel kann einerseits ein bestimmtes Enzym stabilisieren, andererseits ein anderes Enzym destabilisieren (Siehe Torchlin, Martinek, Enzyme Microb. Technol., 1979, Vol. 1, Seite 74). Dies macht die Aufgabe schwierig, ein geeignetes Stabilierungsmittel für eine Enzym-Flüssigformulierung zu finden.

Es ist eine Aufgabe der vorliegenden Erfindung, Enzympräparate für die Lederherstellung auf flüssiger Basis bereitzustellen, wobei sowohl die Aspekte der mikrobiellen Stabilität als auch der Aktivitätskonstanz voll berücksichtigt werden sollen.

Bei der Formulierung der Trägerflüssigkeit hat sich die Erfindung zum Ziel gesetzt, ein möglichst kostengünstiges, ausreichend verfügbares, sowie biologisch gut abbaubares und damit umweltfreundliches Stabilisierungsmittel zu verwenden. Es sollte gut wasserlöslich sein, damit es in hoher Konzentration eingesetzt werden kann.

Eine weitere Aufgabe der Erfindung ist die Vereinigung von verschiedenen Funktionen der Lederbehandlung in einem flüssigen Kombinationspräparat. Nach dem Stand der Technik werden die Ingredienzen für die meist umfangreiche Rezeptur einer Flotte vorwiegend einzeln zugesetzt. Hier ist der Arbeitsaufwand groß und die Gefahr von Dosierungsfehlern hoch.
Alle Maßnahmen, die der Vereinfachung dieser Verfahren und einer Reduzierung der Zahl der zuzusetzenden Mittel dienen, sind ein Fortschritt gegenüber dem Stand der Technik.
Mehrere Funktionen der Lederbehandlung in einem Mittel zu vereinigen, ohne dass dabei die Wirkung der Einzelkomponenten beeinträchtigt wird, bedeutet eine beträchtliche Schwierigkeit. Besonderes Augenmerk muss dabei der Enzymaktivität zugewandt werden, denn gerade Enzyme reagieren besonders empfindlich auf Inhaltsstoffe in der Trägerflüssigkeit. Schwerpunkte für die Inkorporation von anderen Wirkstoffen als den Enzymen sollten dabei Hydrotropika und andere haarlockernde Mittel sein.

### Lösung

Die Aufgabe wurde durch ein Flüssigmittel auf wässriger Basis für die Bearbeitung von Häuten und Fellen in der Wasserwerkstatt, enthaltend an sich bekannte enzymatische Wirkstoffe, gelöst, welches dadurch gekennzeichnet ist,
dass es mindestens 10 Gewichtsprozent bis maximal (60 - x) Gewichtsprozent Melasse enthält, wobei x der Anteil enzymatischer Wirkstoffe in Gewichtsprozent ist. X kann dabei Werte von 0,001 bis 50 Gewichtsprozent annehmen. Bevorzugt liegt die Menge an enzymatischen Wirkstoffen bei 0,1 bis 10 Gewichtsprozent.

Melasse ist ein nachwachsender Rohstoff, biologisch abbaubar und als Abfallstoff sehr kostengünstig. Sie ist der sirupartige, tiefbraune Rückstand der Zucker-Herstellung, der nicht mehr zur Kristallisation gebracht werden kann (Vgl. Kirk-Othmer, Enzyclopedia of Chemical Technology 3rd Ed., Vol. 22, J. Wiley 1985, pp. 514 - 517). Melasse aus Rohrzucker enthält 30-40% Saccharose, 15-25% Invertzucker, bis zu 5% Aconitsäure und kaum Betaine. Der Wassergehalt liegt bei 30-40%.
Melasse aus Rübenzucker enthält laut dem Römpp Chemie Lexikon, (9. Auflage, G. Thieme-Verlag ,1991) im Schnitt 50% Saccharose, 20 % Nichtzuckerstoffe (Dextrine, Betaine, Milchsäure), 2% Stickstoff-Verbindungen, 1% Invertzucker und seltene Zucker wie Raffinose und Kestose, sowie 23 % Wasser. Oft ist die Konzentration an Inhaltsstoffen geringer. Der Wassergehalt liegt dann deutlich höher, bei bis zu 35%.
Melasse ist zwar sehr viskos, kann aber dennoch für sich allein als Trägerflüssigkeit für Enzyme verwendet werden. Zur Herstellung eines derartigen Präparats muss das Enzym, das flüssig oder fest sein kann, direkt in der Melasse gelöst werden. Sofern Enzyme ausnahmsweise rein verfügbar sind, können sie als solche in Melasse eingearbeitet werden. In diesem Fall genügen Mengen von 0,001 bis 0,1 Gewichtsprozent an Enzym, der Rest ist Melasse. Meist sind Enzyme mit verschiedenen Trägerstoffen verschnitten oder in Trägerflüssigkeiten gelöst. Diese müssen bei der Formulierung des erfindungsgemäßen Lederbehandlungspräparates in Kauf genommen werden; sie werden somit zum Bestandteil des Flüssigmittels. Die Zusatzmengen an enzymatischen Wirkstoffen liegen dann im Bereich 0,1 bis 10 Gewichtsprozent.

In der Mehrzahl der Fälle wird man der Melasse Wasser zusetzen, um ein dünnflüssigeres Präparat zu erzeugen. Auch wenn andere Wirkstoffe zugesetzt werden, müssen sie unter Umständen vorher in Wasser gelöst werden, so dass der Endgehalt an Melasse zwangsläufig niedriger liegt. Obwohl Melasse in den meisten Fällen die Hauptträgerflüssigkeit der erfindungsgemäßen Präparate darstellt, kann sie auch in relativ geringer Konzentration enthalten sein, im Extremfall zu nur 10%. Bevorzugt ist sie aber zu 50 bis 80 % im Präparat enthalten. Die restlichen 25 bis 50% bestehen meist aus anderen Ingredienzen, wie Enzymen, verschiedenen Wirkstoffen und Wasser.

Überraschend wurde gefunden, dass Melasse die im Präparat enthaltenen Enzymaktivitäten hervorragend stabilisiert. Das gilt für den Erhalt der Aktivität unmittelbar nach der Formulierung des Flüssigpräparats, als auch bei seiner Lagerung über längere Zeit, z.B. 6 Monate. Für den Fachmann bestanden die oben beschriebenen Vorbehalte, Melasse überhaupt für diesen Zweck einzusetzen. Bestenfalls hätte man einen Stabilisierungseffekt erwarten dürfen, der dem in der Melasse enthaltenen Zuckergehalt entspricht. Von Zuckern, z.B. Saccharose, sind aus dem Stand der Technik Stabilisierungswirkungen bekannt, die vorwiegend auf der Reduzierung der Wasseraktivität fußen. Es stellte sich aber heraus, dass Melasse eine über den Zuckergehalt hinausgehende Stabilisierungswirkung besitzt, die auf der Gegenwart der anderen nicht zuckerartige Inhaltsstoffe beruhen dürfte. Die Verwendung von Melasse als Trägerflüssigkeit in Enzympräparaten ist somit ein wesentliches Kennzeichen der Erfindung.
Dem erfindungsgemäßen Flüssigmittel können die nach dem Stand der Technik üblichen Enzyme einverleibt werden. Aus der großen Zahl der verfügbaren Proteinasen, Lipasen, Amylasen und auch anderen Hydrolasen - die hier mit eingeschlossen werden sollen - kann frei nach Menge und Art gewählt werden. Speziell seien hier bei den Proteinasen die Pankreas-Enzyme, die eigentlich eine Enzymmischung darstellen, Proteinasen aus Bacillus subtilis und B. licheniformis, sowie Aspergillus-Proteinasen genannt, bei den Lipasen die hochalkalische, gentechnisch gewonnene Aspergillusoryzae-Lipase. Die Wahl der Enzymspezies hängt naturgemäß von dem beabsichtigten Einsatzgebiet ab. Soll das Flüssigmittel bevorzugt in der Beize Anwendung finden, wird man Proteinasen oder Lipasen wählen, die ihr pH-Optimum im neutralen bis schwach alkalischen pH-Bereich haben. Für eine universellere Anwendung, nämlich für den Einsatz zusätzlich noch im Äscher und in der Weiche, empfiehlt es sich, Enzyme mit einem pH-Optimum von 9 und darüber einzusetzen. Die Verwendung von Proteinasen oder Lipasen mit einem pH-Optimum =>9 ist eine bevorzugte Ausführungsform der Erfindung. Die im erfindungsgemäßen Flüssigmittel enthaltenen Enzyme können - unter Benutzung des aktuellen Standes der Technik - auch verschiedene unterschiedliche Enzymaktivitäten enthalten, bevorzugt Mischungen von Proteinasen mit Lipasen, wobei beide pH-Optima von =>9 besitzen können, wie in den deutschen Patentanmeldungen DE-A 39 22 748 und DE-A 41 09 826 beschrieben. Es wurde überraschend gefunden, dass in Enzymmischungen in Gegenwart von Melasse die Proteinase andere Enzyme weniger angreift, und in Proteinaselösungen der gefürchtete Selbstverdauungseffekt weitgehend unterdrückt wird.

Proteolytische Enzyme sind im erfindungsgemäßen Präparat meist in einer Aktivität von 100 bis 20000 LVE/g enthalten, die Lipasen in einer Aktivität von 10 - 1000 Lipaseeinheiten/g nach FIP.

Ein weiteres bevorzugtes Kennzeichen der vorliegenden Erfindung ist der Gehalt an Hydrotropika im Flüssigpräparat. Hydrotropika wie Harnstoff wirken in höherer Konzentration auf Proteine denaturierend. Deswegen findet sich in der Patentliteratur ein Schwellenwert von => 1 Mol/l Harnstoff (= 60 g/l = 6 Gew.%) für eine aktivitätsschädigende Beeinflussung des Enzyms. In der vorliegenden Erfindung kann der Gehalt an Hydrotropikum im melassehaltigen Flüssigpräparat auch deutlich höher liegen, nämlich zwischen 3 und 40 Gew. %, speziell zwischen 10 bis 20%. Überraschenderweise wurde auch bei höheren Zusatzmengen an Hydrotropikum kein Aktivitätsverlust beobachtet. Die entsprechend formulierten Flüssigprodukte sind auch nach Lagerung aktivitätsstabil. Bezüglich der Wahl des Hydrotropikums kann - ähnlich wie bei den Enzymspezies - der Stand der der Technik voll genutzt werden. Harnstoff, Guanidinhydrochlorid, Cumolsulfonat und Calciumchlorid sind aber besonders bevorzugt.

Schließlich kann das erfindungsgemäße Flüssigprodukt auch noch andere Wirkstoffe mit dispergierender, schwellungsdämpfender, haarlockernder und kalklösender Wirkung enthalten. Auch in diesem Fall wurden überraschenderweise keine Aktivitätseinbußen des Enzymgehalts beobachtet. Die Zusatzmengen liegen hier zwischen 0.1 bis 20 Gew.%.
Bezüglich der Auswahl dieser Zusätze kann der gesamte Stand der Technik herangezogen werden, wie er weiter oben beschrieben ist. Genannt seien hier stellvertretend für die große mögliche Zahl an Wirkstoffen Polyphosphate als Beispiel für ein kalklösendes Mittel, Natrium-Mercaptoäthanol und Thioglykolsäure als haarlockerndes Mittel, Alkansulfonate und Alkylpolyglykolether als Dispergiermittel, sowie hydroxyfunktionelle Amine als schwellungsdämpfende Mittel.

Diese Wirkstoffe können einzeln dem melassehaltigen Enzymprodukt zugesetzt werden, oder schon gemischt mit einem Hydrotropikum oder mehreren Hydrotropika, aber auch in jeder beliebigen Mischung von Wirkstoffen.
Durch die verschiedenen Zusätze könnte sich in der wässrigen Lösung ein pH-Wert einstellen, der unter Umständen für das Enzym aktivitätsschädigend ist. Das gilt generell für pH-Werte über 12 und unter 4. Da Säure- und Alkalistabilität der jeweils verwendeten Enzyme bekannt sind, wird man den pH-Wert entsprechend anpassen. So wird man z.B. für Bacillus-Proteinasen einen schwach alkalischen pH-Wert (pH = 7-9) im Flüssigprodukt wählen und einen pH-Wert unter 5 vermeiden. In der Mehrzahl der Fälle ist ein pH-Wert zwischen 7 und 9 vorteilhaft für die Enzymaktivität. Die Einstellung des pH-Wertes durch Zugabe von Säuren, Laugen oder Puffer erfolgt zweckmäßig noch vor der Zugabe der Enzyme, um diese nicht einer extremen pH-Belastung aussetzen zu müssen.

Der Wassergehalt im erfindungsgemäßen Flüssigprodukt liegt üblicherweise zwischen 20 und 80 Gew.%, bevorzugt aber bei nur 25 bis 50 Gew.%. Die stabilisierende Wirkung der Melasse kommt bei niedrigen Wassergehalten bzw. hohen Feststoffgehalten besonders zur Geltung. Hohe Feststoffgehalte müssen nicht nur aus den Inhaltsstoffen der Melasse resultieren sondern auch aus den anderen Wirkstoffen, die im Gehalt unter Umständen sogar höher liegen können, als die Inhaltsstoffe der Melasse. In der Mehrzahl der Fälle überwiegen aber letztere. Üblicherweise sind alle Inhaltsstoffe der Flüssigprodukte gelöst. Es ist aber auch möglich, wasserunlösliche Zusätze in der melassehaltigen Lösung zu dispergieren, wenn die Viskosität hoch ist, und dadurch ein Absetzen der Dispersion weitgehend verhindert werden kann. Zur Erhöhung des Feststoffanteils im Flüssigpräparat können zusätzlich auch Salze, z.B. Kochsalz, Ammonium- oder Natriumsulfat, sowie auch andere gut wasserlösliche Stoffe wie Kohlenhydrate, Aminosäuren oder Proteine zugesetzt werden. Ihr Anteil im Präparat wird in der Mehrzahl der Fälle 20 Gew.% nicht übersteigen.

Der hohe Feststoffgehalt bzw. die geringe Wasseraktivität sind nicht nur wichtige Kriterien für die Aktivitätsstabilität sondern auch für die mikrobielle Stabilität. Sie ist bei Feststoffgehalten über 50 Gew.% meist gegeben. Dennoch kann dem Flüssigpräparat ohne weiteres zusätzlich auch ein Konservierungsmittel in der üblichen Menge, vorwiegend < 1%, zugesetzt werden. Das empfiehlt sich auf jeden Fall, wenn der Wassergehalt im Präparat hoch ist, z.B. bei Wassergehalten von über 80%.

Der Einsatz des erfindungsgemäßen Flüssigprodukts erfolgt im allgemeinen vor der jeweiligen Operation der Lederverarbeitung.durch Zugabe zur Flotte. Die Zugabemengen liegen bei 0.1 bis 5 Gew.% bezogen auf das Hautgewicht. 0.5 bis 2 % sind bevorzugt.

### BEISPIELE

Die erfindungsgemäßen Enzympräparationen 1 - 15 sollen dokumentieren, dass
1. der Einsatz von Melasse zu einer höheren Enzymstabilität führt als Saccharose bei etwa gleichem Feststoffgehalt und
2. die verschiedenen Zusätze wie Harnstoff (Hydrotropikum), Mercaptoethanol-Natriumsalz (Haarlockerungsmittel), Diethanolamin als schwellungshemmendes und kalkdispergierendes Mittel keinen oder nur einen untergeordnteten aktivitätsmindernden Einfluss haben (siehe Tabelle 1).
Die Produkte wurden nach folgender Vorschrift hergestellt:

Ein Teil des benötigten Wassers, Stabilisators (Melasse und als Vergleich Saccharose) sowie die entsprechenden Additive (Hydrotropica-, Dispergier-, Emulgierhilfsmittel, Enthaarungsmittel, schwellungsdämpfende Substanzen etc.) werden homogen verrührt. Der pH Wert der Lösung wird mit 2%iger NaOH bzw. 10% Ameisensäure auf einen Wert von ca. 7 eingestellt. Zur Verhinderung des unkontrollierten Wachstums von Mikroorganismen werden 0.1% eines Konservierungsmittels auf Basis von para-Chlor-meta-kresol und einem Isothiazolinonderivat (Mergal KM 80 von Riedel de Haen) zugesetzt. Anschließend wird das Enzym (alkal. Protease aus Bac. subtilis, Pankreatin, Lipase aus Aspergillus oryzae, Pilzprotease aus Aspergillus sojae) zugegeben.Vorteilhafterweise wird das Enzym in wenig Wasser vorher gelöst. Die Enzymmenge wird so bemessen, dass bei Proteasen eine Sollausgangsaktivität von 1000 LVE/g, bei Lipasen eine Aktivität von 100 FIP-Einheiten/g (pH=9) resultiert. Die zugesetzten Mengen an Enzym liegen hier durchwegs unter 1 Gewichtsprozent. Zuletzt wird mit Wasser auf 100 Gewichtsteile (=Gewichts-%) aufgefüllt.

Bemerkung: Bei der eingesetzten Melasse handelt es sich um eine Zuckerrübenmelasse mit einem Zuckeranteil von ca. 40 % Saccharose und einem Wassergehalt von 33%. Ähnliche Ergebnisse werden mit Melassen mit einem Zuckergehalt von 50 % und einem Wassergehalt von 25 % erhalten.

### Stabilitätstests:

Von der frisch bereiteten Enzympräparation wird sofort die Proteinase-Ausgangsenzymaktivität gemessen. Die Probe wird anschließend 7 Tage bei 45 °C gelagert, dann wird die Enzymaktivität neuerlich bestimmt. Die hiebei gemessene Aktivitätsabnahme der Proteinasen entspricht modellhaft einer Lagerung der Enzympräparation 9 Monate lang bei Raumtemperatur. Lipasen sind meist noch stabiler.

Die Ergebnisse sind in Tabelle 1 zusammengefasst. Sie zeigen, dass Melasse die Enzymaktivität besser stabilisiert, als reine Saccharose bei gleichem Feststoffgehalt. Es soll hier angemerkt werden, dass sogar noch bessere Stabilitätseigenschaften bezüglich der Proteinase-Aktivitäten erzielt werden, wenn statt 60 Tl. 75 Tl. Melasse der oben genannten Zusammensetzung eingesetzt werden.

**Tabelle 1**

| | Enzymtyp | Gew.Tl.Stabilis. | Gew.Teil.Additiv 2 | Gew. Additiv 3 | Aktiv.abfall l (%) |
|---|---|---|---|---|---|
| 1 | Alkal. Bakterienprot. | 60 Tl. Melasse | - | | 0,9 |
| 2 | Alkal. Bakterienprot. | 60 Tl. Melasse | 15 Tl. Harnstoff | | 2,1 |
| 3 | Alkal. Bakterienprot. | 40.Tl.Saccharose | | | 58,0 |
| 4 | Alkal. Bakterienprot. | 40 Tl.Saccharose | 15 Tl. Harnstoff | | 28,6 |
| 5 | Alkal. Bakterienprot. | 60 Tl. Melasse | 10 Tl. Mercaptoethanol- Na-Salz | | 10,3 |
| 6 | Alkal. Bakterienprot. | 60 Tl. Melasse | 10 Tl.Diethanolamin | | 6,9 |
| 7 | Alkal. Bakterienprot. | 60 Tl. Melasse | 10 Tl.sulfon.Ölsäure | | 7,8 |
| 8 | Pankreasenzym | 60 Tl. Melasse | | | 66 |
| 9 | Pankreasenzym | 40 Tl.Saccharose | | | 77 |
| 10 | Pilzprotease | 60 Tl. Melasse | | | 46 |
| 11 | Pilzprotease | 40 Tl.Saccharose | | | 49 |
| 12 | Alkal.. Lipase | 60 Tl. Melasse | | | 2,9 |
| 13 | Alkal. Lipase | 40 Tl.Saccharose | | | 19,6 |
| 14 | Alkal. Bakterienprot. | 60 Tl. Melasse | 10 Tl. Mercaptoethanol-Na-Salz | 10 Tl. Diethanolamin | 11,5 |
| 15 | Alkal. Bakterienprot. | 60 Tl. Melasse | 15 Tl. Harnstoff | 10 Tl. sulfon. Ölsäure | 13,5 |

## Patentansprüche

1. Flüssigkeitsmittel auf wässriger Basis für die Bearbeitung von Häuten und Fellen in der Wasserwerkstatt, enthaltend an sich bekannte enzymatische Wirkstoffe, dadurch gekennzeichnet, daß es 10 bis (60-x) Gew.-% Melasse enthält, wobei x der Anteil enzymatischer Wirkstoffe in Gew.-% ist und Werte von 50 - 0,001 annehmen kann.

2. Flüssigmittel entsprechend Anspruch 1
dadurch gekennzeichnet, dass Melasse zu 50 bis 80 Gew.% im Präparat enthalten ist.

3. Flüssigmittel entsprechend Anspruch 1 oder 2,
dadurch gekennzeichnet, dass die Melasse aus der Zuckerrübenverarbeitung stammt.

4. Flüssigmittel entsprechend einem oder mehreren der Ansprüche 1 bis 3,
dadurch gekennzeichnet, dass 3 bis 40, bevorzugt 10 bis 20 Gew.% Hydrotropica enthalten sind.

5. Flüssigmittel entsprechend Anspruch 4,
dadurch gekennzeichnet, dass es eines oder mehrere aus der Gruppe der Hydrotropica ausgewählte Wirkstoffe enthält, nämlich: Harnstoff, Guanidinhydrochlorid, Cumolsulfonat, Calciumchlorid.

6. Flüssigmittel entsprechend einem oder mehreren der Ansprüche 1 bis 5,
dadurch gekennzeichnet, dass es weitere Wirkstoffe mit schwellungsdämpfender, haarlockernder oder kalklösender Wirkung oder eine Mischung zweier oder mehrerer dieser Wirkstoffe enthält.

7. Flüssigmittel entsprechend einem oder mehreren der Ansprüche Anspruch 1 bis 6,
dadurch gekennzeichnet, dass der Wassergehalt bei 20 bis 80, bevorzugt bei 25 bis 50 Gew.% liegt.

8. Flüssigmittel entsprechend einem oder mehreren der Ansprüche 1 bis 7,
dadurch gekennzeichnet, dass der enzymatische Wirkstoff proteolytische Aktivitäten besitzt.

9. Flüssigmittel entsprechend einem oder mehreren der Ansprüche 1 bis 7,
dadurch gekennzeichnet, dass der enzymatische Wirkstoff lipolytische Aktivitäten besitzt.

10. Flüssigmittel entsprechend Anspruch 8 und 9.

11. Flüssigmittel entsprechend Anspruch 8 oder 10,
dadurch gekennzeichnet, dass die proteolytische Aktivität aus einer Bakterienproteinase mit einem pH-Optimum von >9 stammt.

12. Flüssigmittel entsprechend Anspruch 9 oder 10,
dadurch gekennzeichnet, dass die lipolytische Aktivität aus einer Lipase mit einem pH-Optimum von >9 stammt.

13. Flüssigmittel entsprechend einem oder mehreren der Ansprüche Anspruch 1 bis 8 oder 10 bis 11,
dadurch gekennzeichnet, dass es eine proteolytische Aktivität von 100 bis 20000 LVE/g enthält.

14. Verwendung eines Flüssigmittel entsprechend einem oder mehreren der Ansprüche 1 bis 13 in der Wasserwerkstatt zur Verarbeitung von Häuten und Fellen,
dadurch gekennzeichnet, dass es in einer Konzentration von 0.1 bis 5 Gew.%, bevorzugt 0.5 bis 2 % Gew.% bezogen auf das Hautgewicht eingesetzt wird.

## Claims

1. Water-based liquid agent for the processing of hides and skins in the beamhouse, containing enzymatic active substances known *per* se, characterised in that it contains 10 to (60-x) % by weight of molasses, wherein x is the proportion of enzymatic active substances in % by weight and may assume values of from 50 - 0.001.

2. Liquid agent according to claim 1, characterised in that the preparation contains molasses in an amount of from 50 to 80% by weight.

3. Liquid agent according to claim 1 or 2, characterised in that the molasses are obtained from the processing of sugar beet.

4. Liquid agent according to one or more of claims 1 to 3, characterised in that it contains 3 to 40, preferably 10 to 20% by weight of hydrotropics.

5. Liquid agent according to claim 4, characterised in that it contains one or more active substances selected from the group of hydrotropics, namely: urea, guanidine hydrochloride, cumene sulphonate and calcium chloride.

6. Liquid agent according to one or more of claims 1 to 5, characterised in that it contains other active substances with a swelling-attenuating, hair-loosening or lime-releasing activity or a mixture of two or more of these active substances.

7. Liquid agent according to one or more of claims 1 to 6, characterised in that the water content is from 20 to 80, preferably from 25 to 50% by weight.

8. Liquid agent according to one or more of claims 1 to 7, characterised in that the enzymatic active substance has proteolytic activities.

9. Liquid agent according to one or more of claims 1 to 7, characterised in that the enzymatic active substance has lipolytic activities.

10. Liquid agent according to claims 8 and 9.

11. Liquid agent according to claim 8 or 10, characterised in that the proteolytic activity originates from a bacterial proteinase with an optimum pH of >9.

12. Liquid agent according to claim 9 or 10, characterised in that the lipolytic activity originates from a lipase with an optimum pH of >9.

13. Liquid agent according to one or more of claims 1 to 8 or 10 to 11, characterised in that it contains a proteolytic activity of 100 to 20000 LVU/g.

14. Use of a liquid agent according to one or more of claims 1 to 13 in the beamhouse for processing hides and skins, characterised in that it is used in a concentration of from 0.1 to 5% by weight, preferably 0.5 to 2% by weight, based on the weight of the hides.

## Revendications

1. Composition liquide à base aqueuse pour le traitement des peaux et des fourrures dans les ateliers hydrauliques, contenant des principes actifs enzymatiques connus en soi,
caractérisée en ce qu'
elle renferme de 10 à (60-x) % en poids de mélasse dans laquelle x est la proportion de principe actif enzymatique en % en poids et peut prendre des valeurs de 50 à 0,001.

2. Composition liquide selon la revendication 1,
caractérisée en ce que
de la mélasse pour 50 à 80 % en poids est contenue dans la préparation.

3. Composition liquide selon la revendication 1 ou 2,
caractérisée en ce que
la mélasse provient du traitement du sucre de betterave.

4. Composition liquide selon une ou plusieurs des revendications 1 à 3,
caractérisée en ce que
de 3 à 40, de préférence de 10 à 20 % en poids de substance hydrotrope, sont contenus.

5. Composition liquide selon la revendication 4,
caractérisée en ce qu'
elle renferme un ou plusieurs principes actifs choisis dans le groupe des substances hydrotropes notamment l'urée, le chlorhydrate de guanidine, le cumène sulfonate et le chlorure de calcium.

6. Composition liquide selon une ou plusieurs des revendications 1 à 5,
caractérisée en ce qu'
elle renferme un autre principe actif ayant une action qui empêche le gonflement, qui assouplit les poils ou qui dissout le calcaire ou un mélange de deux ou de plusieurs de ces principes actifs.

7. Composition liquide selon une ou plusieurs des revendications 1 à 6,
caractérisée en ce que
la teneur en eau se situe de 20 à 80, de préférence de 25 à 50 % en poids.

8. Composition liquide selon une ou plusieurs des revendications 1 à 7,
caractérisée en ce que
le principe actif enzymatique possède des activités protéolytiques.

9. Composition liquide selon une ou plusieurs des revendications 1 à 7,
caractérisée en ce que
le principe actif enzymatique possède des activités lipolytiques.

10. Composition liquide selon la revendication 8 et 9.

11. Composition liquide selon la revendication 8 ou 10,
caractérisée en ce que
l'activité protéolytique provient d'une protéinase bactérienne ayant un optimum de pH de > 9.

12. Composition liquide selon la revendication 9 ou 10,
caractérisée en ce que
l'activité lipolytique provient d'une lipase ayant un optimum de pH > 9.

13. Composition liquide selon une ou plusieurs des revendications 1 à 8 ou 10 à 11,
caractérisée en ce qu'
elle contient une activité protéolytique allant de 100 à 20.000 LVE/g.

14. Utilisation d'une composition liquide selon une ou plusieurs des revendications 1 à 13 dans un atelier hydraulique en vue du traitement des peaux et des fourrures,
caractérisée en ce qu'
on la met en oeuvre à une concentration allant de 0,1 à 5 % en poids, de préférence de 0,5 à 2 % en poids rapporté au poids de peau.
